# EUROPEAN PATENT SPECIFICATION

(11) **EP 1 237 559 B1**
(45) Date of publication and mention of the grant of the patent: **12.07.2006**
(21) Application number: 00982033.3
(22) Date of filing: 29.11.2000
(51) Int. Cl.: A61K 31/7076, A61K 31/397, A61P 7/02

(54) **PHARMACEUTICAL COMBINATIONS COMPRISING A P2T RECEPTOR ANTAGONIST AND MELAGATRAN**
PHARMAZEUTISCHE ZUSAMMENSTELLUNGEN ENTHALTEND EINEN P2T REZEPTOR ANTAGONIST UND MELAGATRAN
COMBINAISONS PHARMACEUTIQUES AVEC UN ANTAGONISTE DU RECEPTEUR P2T ET MELAGATRAN

(30) Priority: 01.12.1999 SE 9904377
(43) Date of publication of application: 11.09.2002
(60) Divisional application: 06075955.2
(73) Proprietor: AstraZeneca AB, 151 85 Södertälje (SE)
(72) Inventor: DIXON, John, Loughborough, Leics. LE11 5RH (GB); HUMPHRIES, Robert, Loughborough, Leics. LE11 5RH (GB); JARVIS, Gavin, Oxford OX1 3QT (GB); KIRK, Ian, Bakewell RoaAstraZeneca R & D Charnwood (GB)
(74) Representative: Mercer, Christopher Paul
(86) International application number: PCT/SE2000/002378
(87) International publication number: WO 2001/039781

(56) References cited:
- WO-A1-92/17488
- WO-A1-94/18216
- WO-A1-96/16671
- SARAT CCHATTARAJ: 'AR-C69931MX AstraZeneca' CURRENT OPINION CARDIOVASCULAR, PULMONARY & RENAL INVESTIGATIONAL DRUGS vol. 1, no. 5, 1999, pages 600 - 604, XP002937223
- DATABASE CAPLUS [Online] HERBERT J.-M. ET AL.: 'Biochemical and pharmacological properties of clopidogrel: A new ADP receptor antagonist' Retrieved from STN International, accession no. 131:13176 Database accession no. 1999:149238 & EUR. HEART J. SUPPL. vol. 1(SUPPL. A), 1999, pages A31 - A40
- J.J. GARDNER ET AL.: 'Methods for P2T purinoceptor antagonist anti-thrombotic agents and their major metabolite in plasma' METHODOL. SURV. BIOANAL. DRUGS vol. 25, 1998, pages 104 - 111, XP002937225
- ANTHONY H. INGALL ET AL.: 'Antagonists of the platelet P2T receptor. A novel approach to antithrombotic therapy' J. MED. CHEM. vol. 42, 1999, pages 213 - 220, XP002937224

## Description

### FIELD OF THE INVENTION

The present invention relates to pharmaceutical combinations comprising a P_{2*T*} receptor antagonist and another anti-thrombotic agent and to their use in the treatment and prevention of thrombosis.

### BACKGROUND OF THE INVENTION

Increased understanding of the mechanisms underlying thrombosis and of interventions therein has led to a polypharmacological anti-thrombotic approach utilising appropriate combinations of anti-platelet, anti-coagulant and fibrinolytic agents. Examples of anti-thrombotic compounds used include anti-platelet agents such as aspirin, clopidogrel, ticlopidine, GPIIb/IIIa antagonists; anti-coagulants such as thrombin inhibitors, warfarin, heparin and low molecular weight heparins; and fibrinolytic agents including but not limited to, streptokinase, tissue plasminogen activator (tPA) and tenecteplase.

Most patients with acute myocardial infarction are currently treated using either a thrombolytic agent or intervention treatment with percutaneous coronary angioplasty (PTCA). It has been shown that the use of both these methods result in an increase in the number of patients achieving acceptable coronary artery patency at 90 minutes, and that the better the flow in the affected coronary artery, the greater the survival.

However, even the most effective thrombolytic agents with adjunctive aspirin and heparin treatment are only moderately effective in achieving coronary artery patency with normal blood flow (assessed as TIMI grade 3) in about 50% of cases. In addition, in the acute setting where immediate effect is paramount, slow-acting agents leave a window where the patient is not protected from thrombosis. For example, clopidogrel inhibits ADP-induced platelet aggregation and, like the earlier analogue, ticlopidine, has shown clinical efficacy in arterial thrombosis. However, both agents produce incomplete, slow to develop inhibition of the ADP response, properties which are far from ideal in acute therapy, such as prevention of stent occlusion, although increasing use of a loading dose has been a recent advance.

Another short-coming of existing anti-thrombotic agents, and combinations thereof, is that the optimal pharmacodynamic risk:benefit (anti-thrombotic:anti-haemostatic) relationship has not yet been achieved.

Thus there is a need for more effective anti-thrombotic therapy.

Recently it has been shown that P_{2*T*} (also known as P2Y_{ADP} or P2T_{AC}) receptor antagonists offer significant improvements over other anti-thrombotic agents. The P_{2*T*} receptor is primarily involved in mediating platelet aggregation/activation and is a G-protein coupled receptor. The pharmacological characteristics of this receptor have been described, for example, in the references by Humphries et al., Br. J. Pharmacology (1994), 113, 1057-1063, and Fagura et al., Br. J. Pharmacology (1998) 124, 157-164.

International Patent Applications WO 92/17488 and WO 94/18216 disclose novel P_{2T} receptor antagonists and thereof, including compounds of formula (I) (see below). Compound A (a compound of formula (I) wherein R₁ is 3,3,3-trifluoropropyl and R₂ is 2-(methylthio)ethyl) is disclosed in WO 94/18216. Compound B (a compound of formula (I) wherein R₁ is propyl and R₂ is hydrogen) is disclosed in WO 92/17488.

Both compound A and compound B may be used in any condition where platelet activation or aggregation is involved. The compounds may thus act as anti-thrombotic agents and are useful in the treatment or prophylaxis of unstable angina, thromboembolic stroke and peripheral vascular disease. They may also be used in the treatment and prophylaxis of the sequalae of thrombotic complications from angioplasty, thrombolysis, endarterectomy, coronary and vascular graft surgery, renal dialysis and cardio-pulmonary bypass. In addition, they can be used in the treatment and prophylaxis of disseminated intravascular coagulation, deep vein thrombosis, pre-eclampsia, tissue salvage following surgical or accidental trauma, vasculitis, arteritis, thrombocythaemia, ischemia and migraine.

### DISCLOSURE OF THE INVENTION

The inventors of the present invention have surprisingly found that administration of a combination of a P_{2*T*} receptor antagonist or a pharmaceutically acceptable derivative thereof, and another anti-thrombotic agent or a pharmaceutically acceptable derivative thereof, offers a significant improvement over other anti-thrombotic treatments.

Accordingly, the combined administration of a P_{*2T*} receptor antagonist or a pharmaceutically acceptable derivative thereof and another anti-thrombotic agent or a pharmaceutically acceptable derivative thereof, can be used in the treatment and prevention of thrombosis, particularly in the treatment of the thrombotic complications of atherosclerotic disease and interventions therein.

According to a first aspect of the invention there is provided a kit of parts comprising:
(a) a P_{2*T*} receptor antagonist of the formula (I): 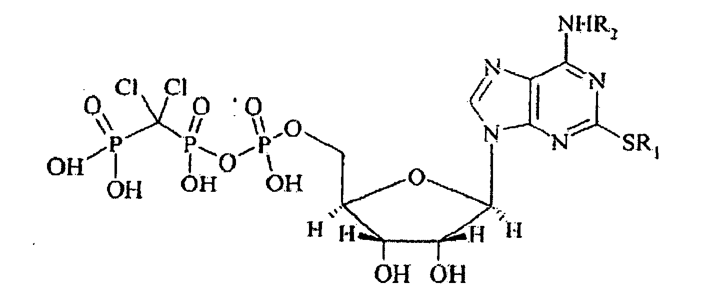 wherein:
   R₁ is 3,3,3-trifluoropropyl and R₂ is 2-(methylthio)ethyl,
   or a pharmaceutically acceptable derivative thereof; and
(b) another antithrombotic agent selected from melagatran or a prodrug thereof;
where components (a) and (b) are each provided in a form (which may be the same or different) that is suitable for administration in conjunction with each other.

According to a further aspect of the invention there is provided a pharmaceutical formulation comprising:
(a) *P*_{*2T*} receptor antagonist of the formula (I): 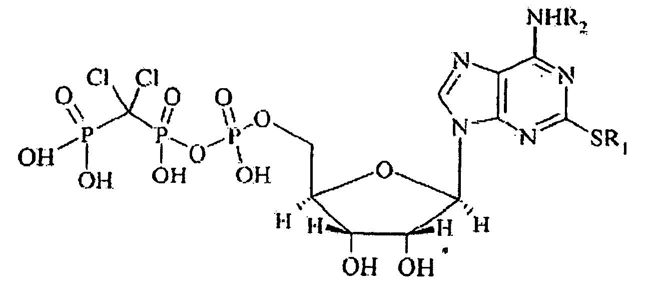 wherein:
   R₁ is 3,3,3-trifluoropropyl and R₂ is 2-(methylthio)ethyl,
   or a pharmaceutically acceptable derivative thereof; and
(b) another antithrombotic agent selected from melagatran or a prodrug thereof;
in admixture with a pharmaceutically acceptable adjuvant, diluent or carrier.

Pharmaceutically acceptable derivatives of a P_{*2T*} receptor antagonist and other antithrombotic agent include salts (e.g. pharmaceutically acceptable non-toxic organic or inorganic acid addition salts (such as a salt of hydrochloric, hydrobromic, nitric, sulphuric or acetic acid)), solvates and solvates of salts.

If more than one formulation comprising component (a) or component (b) is present, for example in order to provide for repeat dosing, such formulations may be the same, or may be different in terms of the dosage, chemical composition and/or physical form.

Examples of direct thrombin inhibitors include melagatran (WO94/29336). Prodrugs of melagatran include those described in WO 97/23499, and particularly include Example 17 of that application. Example 17 of WO 97/23499 is H 376, which is EtO₂C-CH₂-(R)Cgl-Aze-Pab-OH, wherein Cgl is cyclohexylglycinyl, Aze is (S)-azetidine-2-carbonyl and Pab is para-amidinobenzylamino and the OH replaces one of the amidino hydrogens in Pab.

In accordance with the invention, the P_{*2T*} receptor antagonist, other anti-thrombotic agent, and derivatives of either, may be administered orally, intravenously, subcutaneously, buccally, rectally, dermally, nasally, tracheally, bronchially, topically, or via inhalation into the lung. Preferred modes of delivery are systemic. For the P_{*2T*} receptor antagonist and derivatives thereof, preferred modes of administration are parenteral, more preferably intravenous. For the other anti-thrombotic agent, preferred modes of administration are oral or, in the case of unfractionated or low molecular weight heparins, certain direct thrombin inhibitors and fibrinolytic agents, intravenous or subcutaneous.

The sequence in which the formulations comprising the P_{*2T*} receptor antagonist and the other anti-thrombotic agent may be administered (i.e. whether, and at what point, sequential, separate and/or simultaneous administration takes place) may be determined by the physician or skilled person. For example, the sequence may depend upon many factors, such as whether, at any time during the course or period of treatment, one or other of the formulations cannot be administered to the person for practical reasons (e.g. the person is unconscious and thus unable to take an oral formulation).

Respective formulations comprising component (a) and/or component (b) may be administered, sequentially, separately and/or simultaneously, over the course of treating the relevant condition, which condition may be acute or chronic. Preferably, the two formulations are administered (optionally repeatedly) sufficiently closely in time for there to be a beneficial effect for the patient, that is greater, over the course of the treating the relevant condition, than if either of the two formulations are administered (optionally repeatedly) alone, in the absence of the other formulation, over the same course of treatment. Determination of whether a combination provides a greater beneficial effect in respect of, and over the course of treatment of a particular condition, will depend upon the condition to be treated or prevented, but may be achieved routinely by the skilled person.

Alternatively, one or other of the two component formulations may be administered (optionally repeatedly) prior to, after, and/or at the same time as, administration with the other component. Individual doses of a P_{*2T*} receptor antagonist and other anti-thrombotic agent may be used within 48 hours (e.g. 24 hours) of each other.

In the therapeutic treatment of mammals, and especially humans, the P_{*2T*} receptor antagonist, other anti-thrombotic agent, and derivatives of either, may be administered alone, but will generally be administered as a pharmaceutical formulation in admixture with a pharmaceutically acceptable adjuvant, diluent or carrier, which should be selected with due regard to the intended route of administration and standard pharmaceutical practice.

In accordance with the invention, the kit of parts may be used in medical therapy, suitably in the treatment of thrombosis. The treatment of thrombosis will be understood by those skilled in the art to include the treatment and prevention of thrombotic complications of atherosclerotic disease and interventions therein, such as fibrinolysis, endarterectomy or percutaneous transluminal coronary revascularisation (PTCR), including, but not limited to, percutaneous transluminal coronary angioplasty (PTCA) with or without stenting. Thrombotic complications of atherosclerotic disease include, but are not limited to, acute coronary syndrome (encompassing acute myocardial infarction with or without ST elevation and unstable angina) and thrombotic stroke.

The present invention may be used for treating thrombosis (for example thrombotic complications of atherosclerotic disease and interventions therein, such as fibrinolysis, endarterectomy or percutaneous transluminal coronary revascularisation (PTCR), including, but not limited to, percutaneous transluminal coronary angioplasty (PTCA) with or without stenting) which comprises using a kit of parts for administering a therapeutically effective amount of the P_{*2T*} receptor and another anti-thrombotic agent to a person suffering from or susceptible to such a disorder.

For avoidance of doubt the term "treatment" includes therapeutic and/or prophylactic treatment.

Preferably component component (a) is administered parenterally prior to surgery and component (b) is administered orally following that surgery.

According to another aspect of the invention, there is provided a method of making a kit of parts as defined herein, which comprises bringing a component (a) into association with a component (b) thus rendering the two components suitable for administration in conjunction with each other. By bringing the two components into association with each other, we include that components (a) and (b) may be:
i) packaged presented and purchased as separate formulations which are subsequently used in conjunction in combination therapy; or
ii) packaged and presented together as separate components of a combination pack for use in conjunction with each other in combination therapy.

The present invention still further provides a kit of parts comprising:
(1) components (a) and (b) as defined herein; together with
(2) instructions to use the components in conjunction with each other.

The invention further provides the use of the P_{*2T*} receptor antagonist, or a pharmaceutically acceptable derivative thereof, in the manufacture of a kit of parts for the treatment of thrombosis.

Components (a) and (b) as described herein may also be co-formulated as a combined preparation (i.e. presented as a single formulation including the P_{*2T*} receptor antagonist and other anti-thrombotic agent).

The present invention provides a pharmaceutical formulation comprising:
(a) the P_{2*T*} receptor antagonist or a pharmaceutically acceptable derivative thereof; and
(b) another anti-thrombotic agent selected from melagatran or a prodrug thereof in admixture with a pharmaceutically acceptable adjuvant, diluent or carrier;
for use in medical therapy, suitably in the treatment of thrombosis.

In another aspect of the present invention, there is provided a process for the preparation of a phannaceulical formulation which comprises mixing the P_{*2T*} receptor antagonist with another anti-thrombotic agent selected from melagatran or a produg thereof.

The invention further provides the use of a pharmaceutical formulation as hereinbefore defined in the manufacture of a medicament for the treatment of thrombosis.

Another aspect of the invention involves the use of:
(a) a pharmaceutical formulation comprising the P_{*2T*} receptor antagonist or a pharmaceutically acceptable derivative thereof, in admixture with a pharmaceutically acceptable adjuvant, diluent or carrier; and
(b) a pharmaceutical formulation comprising another anti-thrombotic agent selected from melagatran on a prodrug thereof, in admixture with a pharmaceutically acceptable adjuvant, diluent or carrier,
in therapy, suitably in the treatment of thrombosis.

In another aspect of the present invention, there is provided the use of the P_{*2T*} receptor antagonist, or a pharmaceutically acceptable derivative thereof, in the manufacture of a medicament to be used in combination with the other anti-thrombotic agent in the treatment of thrombosis.

Suitable formulations for administering a P_{*2T*} receptor antagonist are known in the art, and include those known from WO 92/17488 and WO 94/18216.

Suitable formulations for administering other anti-thrombotic agent are described in the literature, for example, for melagatran, or a prodrug of melagatran, suitable formulations include those described in *inter alia* WO 94/29336, WO 96/14084, WO 96/16671, WO 97/23499, WO 97/39770, WO 97/45138, WO 98/16252, WO 99/27912, WO 99/27913, WO 00/13672 and WO 00/12043. Otherwise, the preparation of suitable formulations may be achieved by the skilled person using routine techniques.

Suitable doses of the P_{2*T*} receptor antagonist, the other anti-thrombotic agent, and derivatives of either can be determined by the medical practitioner or other skilled person, and will depend on the severity of the condition, and on the person to be treated, as well as the compound(s) which is/are employed. Respective doses are discussed in the prior art documents disclosing P_{*2T*} receptor antagonists and other anti-thrombotic agents that are mentioned hereinbefore.

In the case of a compound of formula (I), suitable doses of active compound in the therapeutic and/or prophylactic treatment of mammalian, especially human, patients include those which give a mean plasma concentration of up to 5 µmol/L, for example in the range 0.001 to 5 µmol/L over the course of treatment of the relevant condition. In any event, the physician, or the skilled person, will be able to determine the actual dosage which will be most suitable for an individual person, which is likely to vary with the condition that is to be treated, as well as the age, weight, sex and response of the particular person to be treated. The above-mentioned dosages are exemplary of the average case. There can, of course, be individual instances where higher or lower dosage ranges are merited, and such are within the scope of this invention.

The pharmaceutical formulation of the invention may, and indeed will usually, contain various other ingredients known in the art, for example preservatives, stabilising agents, viscosity-regulating agents, emulsifying agents or buffering agents. Thus the pharmaceutical formulation of the invention will typically comprise a total amount of (a) the P_{2*T*} receptor antagonist and (b) the other anti-thrombotic agent (the active ingredients) in the range from 0.05 to 99 %w (per cent by weight), more preferably in the range from 0.10 to 70 %w, and even more preferably in the range from 0.10 to 50 %w, all percentages by weight being based on total formulation.

### EXAMPLES

The invention is illustrated by the following examples, utilising compound A (a compound of formula (I) wherein R₁ is 3,3,3-trifluoropropyl and R₂ is 2-(methylthio)ethyl).

### Example 1

### Canine Coronary Thrombosis Model - compound A and aspirin/heparin

Compound A was used in combination with aspirin and unfractionated heparin in a dog model of coronary artery thrombosis to determine whether addition of a P_{*2T*}-receptor antagonist to these standard anti-platelet and anti-coagulant agents could improve coronary artery patency after thrombolysis with tPA. All animals were treated with both aspirin 325 mg and unfractionated heparin 80 U/kg then 17 U/kg/h. The test group (n = 10) was treated with compound A (4 µg/kg/min iv) from 10 min prior to tPA until end of protocol (2 h post reperfusion). The placebo group (n = 10) received only a saline infusion iv from 10 min prior to tPA until end of protocol (2 h post reperfusion).

The results of the experiments are evident in tables 1 and 2.

Coronary artery blood flow following successful thrombolysis with tPA was significantly better maintained in a group of animals receiving compound A in addition to aspirin and heparin than in a group receiving saline, aspirin and heparin (table 1).

**Table 1. Effects on coronary thrombolysis by tPA**

| **Parameter** | **Saline** | **Compound A** |
|---|---|---|
| Baseline blood flow (ml/min) | 65.3 ± 7.5 | 62.3 ± 8.5 ns |
| Time to occlusion (min) | 55.5 ± 14.0 | 62.3 ± 14.7ns |
| Reperfusion rate | 100% | 100%ns |
| Time to reflow (min) | 21.5 ± 2.9 | 20 ± 6.1ns |
| Reflow duration (min) | 75.0 ± 39.9 | 119.7±0.7* |
| Cyclic flow variation | 50% | 0%* |
| Reocclusion | 60% | 0%* |

| | | |
|---|---|---|
| * P<0.05 | | |

Infarct size was also reduced significantly (P < 0.05) in animals receiving compound A (table 2). These results suggest that significant additional clinical benefit will be attained when a P_{*2T*} antagonist is combined with a fibrinolytic agent and standard anti-platelet and anti-coagulant therapy.

**Table 2. Infarct size reduction**

| Saline | | Compound A | |
|---|---|---|---|
| Area at risk (cm²) | 48.7 ±6.9 | 49.9 ± 8.4 | Ns |
| Infarct size (cm²) | 9.3 ± 4.4 | 4.7 ± 4.7 | P = 0.034 |

### Example 2

### Human blood in vitro - compound A and clopidogrel

Compound A (500 nM final concentration) was added to blood from healthy human volunteers receiving clopidogrel (Sanofi-Winthrop, 75 mg/day for 11 days). ADP-induced platelet aggregation (+/- compound A) was measured using whole blood impedance aggregometry.

Clopidogrel alone resulted in slowly developing, incomplete inhibition of the ADP response (Table 3). Compound A added *in vitro* produced complete or near complete inhibition of the response to low to intermediate concentrations of ADP (up to 30 µM) both before and during administration of clopidogrel (data for ADP 10 µM shown in Table 3) while substantial inhibition of the response to the highest concentration of ADP used (300 µM) required a combination of both compound A and clopidogrel.

**Table 3: Effect of oral clopidogrel (75 mg/day) on ADP (10 and 300 µM)-induced platelet aggregation measured in blood from healthy human volunteers ex vivo (+/compound A (500 nM) added in vitro)**

| Duration of clopidogrel admirustration (days) | Aggregation (ohms) (mean ± SD, n = 7 - 8 except where indicated) | | | |
|---|---|---|---|---|
| | ADP 10 µM | | ADP 300 µM | |
| | - compound A | + compound A | - compound A | + compound A |
| 0 | 14.9 ± 1.9 | 0.5 ± 0.7 | Not measured | 6.5 ± 3.4 |
| 1 | 13.8 ± 2.3 | 0.4 ± 0.7 | Not measured | 4.2 ± 2.6 |
| 2 | 11.8 ± 3.4 | 0.4 ± 0.6 | Not measured | 2.9 ± 2.3 |
| 3 | 10.2±4.5 | 0.6±0.7 | 13.9⁽ⁿ⁼¹⁾ | 2.7±2.1 |
| 11 | 8.2±4.4 | 0.6±0.8 | 11.4±5.2⁽ⁿ⁼³⁾ | 2.8 ±2.8 |

### Abbreviations

ADP = adenosine diphosphate
GPIIb/IIIa antagonist = glycoprotein IIb/IIIa antagonist
PTCR = percutaneous transluminal coronary revascularisation
PTCA = percutaneous transluminal coronary angioplasty
TIMI = thrombolysis in myocardial infarction
tPA = tissue plasminogen activator

## Claims

1. A kit of parts comprising:
(a) a P_{2*T*} receptor antagonist of the formula (I): 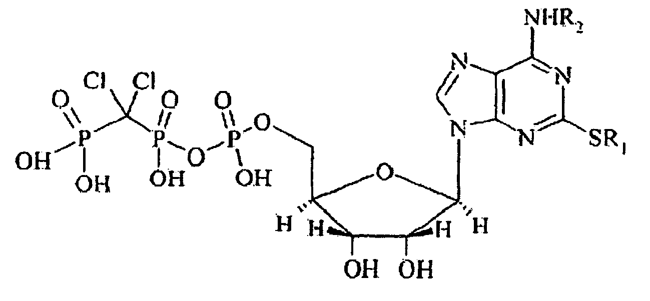 wherein:
R₁ is 3,3,3-trifluoropropyl and R₂ is 2-(methylthio)ethyl,
or a pharmaceutically acceptable derivative thereof; and
(b) another antithrombotic agent selected from melagatran or a prodrug thereof;
where components (a) and (b) are each provided in a form (which may be the same or different) that is suitable for administration in conjunction with each other.

2. A kit of parts as claimed in claim 1, wherein the pharmaceutically acceptable derivative of the P_{*2T*} receptor antagonist of formula (I) is selected from salts, solvates and solvates of salts.

3. A kit of parts as claimed in claim 1 or claim 2, wherein the pharmaceutically acceptable derivative of the P_{*2T*} receptor antagonist of formula (I) is a pharmaceutically acceptable non-toxic organic or inorganic acid addition salt.

4. A kit of parts as claimed in any preceding claim, wherein the prodrug of melagatran is EtO₂C-CH₂-(R)Cgl-Aze-Pab-OH.

5. A kit of parts according to any preceding claim, wherein components (a) and (b) are suitable for sequential, separate and/or simultaneous administration.

6. A kit of parts according to any of claims 1 to 5, for use in medical therapy.

7. A kit of parts according to any of claims 1 to 5, for use in the treatment of thrombosis.

8. Use of a P_{*2T*} receptor antagonist of the formula (I) and melagatran or a prodrug thereof according to any of claims 1 to 5, in the manufacture of a medicament, for the treatment of a person suffering from or susceptible to thrombosis.

9. Use according to claim 8, wherein the P_{*2T*} receptor antagonist of the formula (I) is administered parenterally prior to surgery and the prodrug of melagatran is administered orally following that surgery.

10. The use of a P_{*2T*} receptor antagonist according to any one of claims 1 to 9, or a pharmaceutically acceptable derivative thereof, in the manufacture of a kit of parts for the treatment of thrombosis.

11. A pharmaceutical formulation comprising:
(a) P_{*2T*} receptor antagonist of the formula (I): 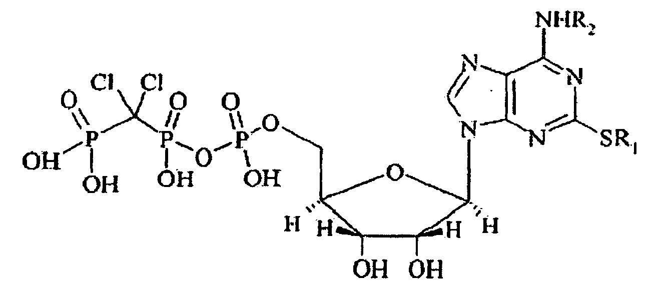 wherein:
R₁ is 3,3,3-trifluoropropyl and R₂ is 2-(methylthio)ethyl,
or a pharmaceutically acceptable derivative thereof; and
(b) another antithrombotic agent selected from melagatran or a prodrug thereof;
in admixture with a pharmaceutically acceptable adjuvant, diluent or carrier.

12. The pharmaceutical formulation according to claim 11, wherein the pharmaceutically acceptable derivative of the P_{*2T*} receptor antagonist of formula (I) is selected from salts, solvates and solvates of salts.

13. The pharmaceutical formulation according to claim 11 or claim 12, wherein the pharmaceutically acceptable derivative of the P_{*2T*} receptor antagonist of formula (I) is a pharmaceutically acceptable non-toxic organic or inorganic acid addition salt.

14. A pharmaceutical formulation according to any one of claims 11 to 13 for use in medical therapy.

15. A pharmaceutical formulation according to any one of claims 11 to 13 for use in the treatment of thrombosis.

16. The use of a pharmaceutical formulation according to any one of claims 11 to 13 in the manufacture of a medicament for the treatment of thrombosis.

17. A process for the preparation of a pharmaceutical formulation according to any one of claims 11 to 13 which comprises mixing the P_{*2T*} receptor antagonist with melagatran or a prodrug thereof.

## Patentansprüche

1. Kit aus Teilen, umfassend
(a) einen P_{2*T*}-Rezeptorantagonisten der Formel (I): 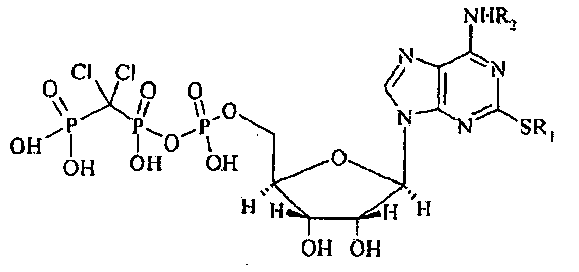 wobei
R₁ 3,3,3-Trifluorpropyl ist und R₂ 2-(Methylthio)ethyl ist,
oder ein pharmazeutisch verträgliches Derivat davon; und
(b) ein weiteres antithrombotisches Mittel, ausgewählt aus Melagatran und einem Prodrug davon;
wobei die Komponenten (a) und (b) jeweils in einer Form (die gleich oder verschieden sein kann), die zur Verabreichung in Verbindung miteinander geeignet ist, bereitgestellt sind.

2. Kit aus Teilen nach Anspruch 1, wobei das pharmazeutisch verträgliche Derivat des P_{2*T*}-Rezeptorantagonisten der Formel (I) aus Salzen, Solvaten und Solvaten von Salzen ausgewählt ist.

3. Kit aus Teilen nach Anspruch 1 oder Anspruch 2, wobei das pharmazeutisch verträgliche Derivat des P_{2*T*}-Rezeptorantagonisten der Formel (I) ein pharmazeutisch verträgliches nicht toxisches, organisches oder anorganisches Säure-Additionssalz ist.

4. Kit aus Teilen nach einem vorhergehenden Anspruch, wobei das Melagatran-Prodrug EtO₂C-CH₂-(R)Cgl-Aze-Pab-OH ist.

5. Kit aus Teilen nach einem vorhergehenden Anspruch, wobei die Komponenten (a) und (b) zur sequenziellen, separaten oder gleichzeitigen Verabreichung geeignet sind.

6. Kit aus Teilen nach einem der Ansprüche 1 bis 5 zur Verwendung in der medizinischen Therapie.

7. Kit aus Teilen nach einem der Ansprüche 1 bis 5 zur Verwendung bei der Behandlung von Thrombose.

8. Verwendung eines P_{2T}-Rezeptorantagonisten der Formel (I) und von Melagatran oder eines Prodrug davon, nach einem der Ansprüche 1 bis 5 bei der Herstellung eines Medikaments zur Behandlung einer Person, die an Thrombose leidet oder Thrombose-empfindlich ist.

9. Verwendung nach Anspruch 8, wobei der P_{*2T*}-Rezeptorantagonist der Formel (I) vor einem operativen Eingriff parenteral verabreicht wird und das Melagatran-Prodrug nach diesem operativen Eingriff oral verabreicht wird.

10. Verwendung eines P_{2T}-Rezeptorantagonisten nach einem der Ansprüche 1 bis 9 oder eines pharmazeutisch verträglichen Derivats davon bei der Herstellung eines Kit aus Teilen zur Behandlung von Thrombose.

11. Pharmazeutische Formulierung, umfassend:
(a) einen P_{2*T*}-Rezeptorantagonisten der Formel (I), 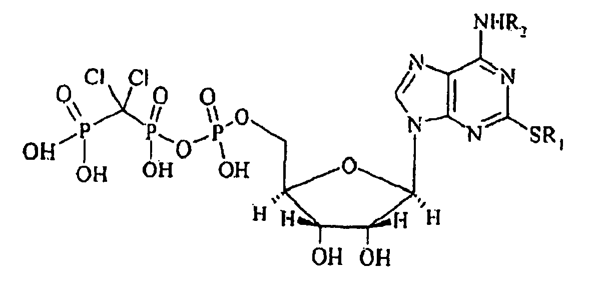 wobei
R₁ 3,3,3-Trifluorpropyl ist und R₂ 2-(Methylthio)ethyl ist,
oder ein pharmazeutisch verträgliches Derivat davon; und
(b) ein weiteres antithrombotisches Mittel, ausgewählt aus Melagatran und einem Prodrug davon;
im Gemisch mit einem pharmazeutisch verträglichen Adjuvans, Verdünnungsmittel oder Träger.

12. Pharmazeutische Formulierung nach Anspruch 11, wobei das pharmazeutisch verträgliche Derivat des P_{2T}-Rezeptorantagonisten der Formel (I) aus Salzen, Solvaten und Solvaten von Salzen ausgewählt ist.

13. Pharmazeutische Formulierung nach Anspruch 11 oder Anspruch 12, wobei das pharmazeutisch verträgliche Derivat des P_{2T-}Rezeptorantagonisten der Formel (I) ein pharmazeutisch verträgliches nicht toxisches organisches oder anorganisches Säure-Additionssalz ist.

14. Pharmazeutische Formulierung nach einem der Ansprüche 11 bis 13 zur Verwendung in der medizinischen Therapie.

15. Pharmazeutische Formulierung nach einem der Ansprüche 11 bis 13 zur Verwendung bei der Behandlung von Thrombose.

16. Verwendung einer pharmazeutischen Formulierung nach einem der Ansprüche 11 bis 13 bei der Herstellung eines Medikaments zur Behandlung von Thrombose.

17. Verfahren zur Herstellung einer pharmazeutischen Formulierung nach einem der Ansprüche 11 bis 13, das das Mischen des P_{*2T-*}Rezeptorantagonisten mit Melagatran oder einem Prodrug davon umfasst.

## Revendications

1. Ensemble de composants comprenant :
(a) un antagoniste du récepteur P_{2*T*} de formule (I) : 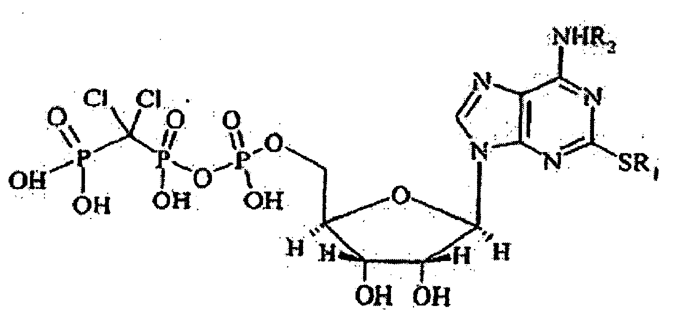 dans laquelle :
R₁ est le groupe 3,3,3-trifluoropropyle et R₂ est le groupe 2-(méthylthio)éthyle,
ou un dérivé pharmaceutiquement acceptable de celui-ci ; et
(b) un autre agent antithrombotique choisi parmi le mélagatran ou un précurseur de médicament de celui-ci ;
où les composants (a) et (b) sont chacun fournis sous une forme (qui peut être identique ou différente) qui est appropriée pour une administration en combinaison l'un avec l'autre.

2. Ensemble de composants tel que revendiqué dans la revendication 1, dans lequel le dérivé pharmaceutiquement acceptable de l'antagoniste du récepteur P_{*2T*} de formule (I) est choisi parmi les sels, les solvates et les solvates de sels.

3. Ensemble de composants tel que revendiqué dans la revendication 1 ou la revendication 2, dans lequel le dérivé pharmaceutiquement acceptable de l'antagoniste du récepteur P_{2*T*} de formule (I) est un sel d'addition acide organique ou inorganique non toxique pharmaceutiquement acceptable.

4. Ensemble de composants tel que revendiqué dans une quelconque revendication précédente, dans lequel le précurseur de médicament du mélagatran est EtO₂C-CH₂-(R)Cgl-Aze-Pab-OH.

5. Ensemble de composants tel que revendiqué dans une quelconque revendication précédente, dans lequel les composants (a) et (b) sont appropriés à une administration séquentielle, séparée et/ou simultanée.

6. Ensemble de composants selon l'une quelconque des revendications 1 à 5, destiné à être utilisé dans une thérapie médicale.

7. Ensemble de composants selon l'une quelconque des revendications 1 à 5, destiné à être utilisé dans le traitement de la thrombose.

8. Utilisation d'un antagoniste du récepteur P_{*2T*} de formule (I) et de mélagatran ou d'un précurseur de médicament de celui-ci selon l'une quelconque des revendications 1 à 5, dans la fabrication d'un médicament, pour le traitement d'une personne souffrant ou susceptible de souffrir d'une thrombose.

9. Utilisation selon la revendication 8, dans laquelle l'antagoniste du récepteur P_{*2T*} de formule (I) est administré par voie parentérale avant l'intervention chirurgicale et le précurseur de médicament du mélagatran est administré par voie orale après cette intervention chirurgicale.

10. Utilisation d'un antagoniste du récepteur *P*_{*2T*} selon l'une quelconque des revendications 1 à 9, ou d'un dérivé pharmaceutiquement acceptable de celui-ci, dans la fabrication d'un ensemble de composants pour le traitement de la thrombose.

11. Formulation pharmaceutique comprenant :
(a) un antagoniste du récepteur P_{2*T*} de formule (I) : 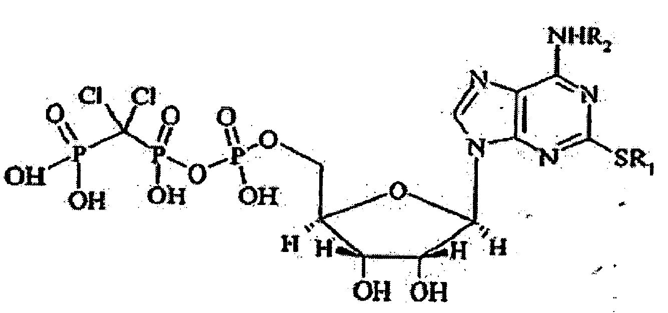 dans laquelle :
R₁ est le groupe 3,3,3-trifluoropropyle et R₂ est le groupe 2-(méthylthio)éthyle,
ou un dérivé pharmaceutiquement acceptable de celui-ci ; et
(b) un autre agent antithrombotique choisi parmi le mélagatran ou un précurseur de médicament de celui-ci ;
en mélange avec un adjuvant, un diluant ou un véhicule pharmaceutiquement acceptable.

12. Formulation pharmaceutique selon la revendication 11, dans laquelle le dérivé pharmaceutiquement acceptable de l'antagoniste du récepteur P_{*2T*} de formule (I) est choisi parmi les sels, les solvates et les solvates de sels.

13. Formulation pharmaceutique selon la revendication 11 ou la revendication 12, dans laquelle le dérivé pharmaceutiquement acceptable de l'antagoniste du récepteur P_{*2T*} de formule (I) est un sel d'addition acide organique ou inorganique non toxique pharmaceutiquement acceptable.

14. Formulation pharmaceutique selon l'une quelconque des revendications 11 à 13, destinée à être utilisée dans une thérapie médicale.

15. Formulation pharmaceutique selon l'une quelconque des revendications 11 à 13, destinée à être utilisée dans le traitement de la thrombose.

16. Utilisation d'une formulation pharmaceutique selon l'une quelconque des revendications 11 à 13 dans la fabrication d'un médicament pour le traitement de la thrombose.

17. Procédé de préparation d'une formulation pharmaceutique selon l'une quelconque des revendications 11 à 13 qui comprend l'étape consistant à mélanger l'antagoniste du récepteur P_{*2T*} avec du mélagatran ou un précurseur de médicament de celui-ci.
